# EUROPEAN PATENT SPECIFICATION

(11) **EP 4 029 462 B1**
(45) Date of publication and mention of the grant of the patent: **06.12.2023**
(21) Application number: 21305056.0
(22) Date of filing: 19.01.2021
(51) Int. Cl.: A61B 17/34, A61B 90/11, A61B 17/00, A61B 90/00

(54) **STAND FOR A PUNCTURING DEVICE**
STÄNDER FÜR EINE PUNKTIONSVORRICHTUNG
SUPPORT POUR UN DISPOSITIF DE PERFORATION

(43) Date of publication of application: 20.07.2022
(73) Proprietor: IMACTIS, 38400 Saint-Martin-d'Hères (FR)
(72) Inventor: CARRAT, Lionel, 38400 Saint Martin d'Heres (FR); CLAVERIE, Frédéric, 38180 Seyssins (FR)
(74) Representative: Regimbeau

(56) References cited:
- WO-A1-02/36032
- CN-A- 104 546 078
- US-A- 5 280 427
- US-A- 5 575 798
- US-A1- 2012 184 956
- US-A1- 2012 265 098
- US-A1- 2017 014 200
- US-A1- 2017 311 978

## Description

### TECHNICAL FIELD

The present invention relates generally to means for supporting medical instruments being inserted into a patient's body.

More precisely the present invention relates to a stand for a puncturing device.

### BACKGROUND ART

During percutaneous interventions, imaging systems such as Magnetic resonance imaging (MRI), Computerized tomography (CT), or X-rays systems are used to provide images of the patient's internal organs.

These imaging systems usually provide doctors with a cross-sectional picture of a patient's internal organs and tissues; and if a puncturing operation is to be performed, the imaging systems provide the doctor with image data from which the doctor determines the puncturing position, the direction and the depth for a puncturing device so as to reach the target site.

While inserting the puncturing device, e.g. a puncturing needle, the doctor may wish to control that the puncturing position, the direction and the depth of the puncturing device are still in accordance with what he or she has planned.

Therefore, the doctor will perform a control imaging while the puncturing device is inserted into the patient's body.

However, some puncturing devices, like puncturing needles, tend to tilt because of their own height and/or weight if they are inserted of only a small depth into the patient's body. If it occurs, the control image is not representative of the needle trajectory and the control image is not usable.

Doctors may use adhesives, pads, compresses or other devices to try to lock the puncturing device in position while the control imaging is performed.

Obviously, these fixation techniques are not satisfactory. Indeed, adhesives or pads may come loose, and compresses may themselves tilt, while the control imaging is performed. And, again, the control image is then not usable.

On the other hand, puncturing guides for guiding a puncturing needle into a target site within a patient's body have already been provided. An example of such puncturing guides is disclosed in document EP 1 534 153. Whilst these puncturing guides aim at both placing and adjusting the entrance angle of the puncturing needle, they may also function as stands to ensure the needle do not tilt during a control imaging.

However, these puncturing guides are bulky. Therefore, it is impossible to insert several puncturing devices at the same place or in the same area, close one to the other, on the patient's body.

Besides, these puncturing guides are fixedly mounted on the puncturing site during the entire puncture procedure, which limits the doctor's freedom of movement at specific steps of the procedure. Indeed, the doctor needs to withdraw the puncturing device from the patient's body in order to withdraw the puncturing guide from the puncturing site.

Additionally, when using these puncturing guides, one must first choose an entry point on the patient's skin for the puncturing device to penetrate. And, although such puncturing guides will allow the angle of the puncturing device to change during the imaging process, the entry point will remain the same, which may be a drawback if the doctor wishes to adjust the target site within the patient's body.

Last, these puncturing guides are set for specific properties of the puncturing devices (e.g. specific puncturing needle diameters). Therefore, they are very difficult to use when different puncturing devices are needed (e.g. an anesthetic needle and a biopsy gun).

Document US 5,280,427 discloses an apparatus for guiding the needle of a tissue sampling device to a target location within the body of a patient. The device permits accurate and easy retrieval of acceptable tissue specimens from locations within the body of a patient which require angling of the needle to reach the target tissue. The apparatus further permits accurate and easy retrieval of tissue specimens from even small pathological changes, and the apparatus can be introduced into the tomograph with the patient to verify correct location of the biopsy needle with respect to the target tissue. The device not only directs the biopsy needle along the desired path but also controls the depth of penetration of the needle to prevent accidental overpenetration of the needle. The guidance device is not limited to the plane normal to the longitudinal axis of the patient but is capable of guiding the needle in a plane which is oblique to the longitudinal axis.

Document US 2012/184956 discloses apparatus and methods for use in an image-guided interventional procedure are described herein. In one aspect of the disclosure of this document, an apparatus includes a base configured to be releasably coupled to a patient's skin. A support portion extends from the base at an angle transverse to a longitudinal axis defined by the base. The support portion has a first end portion and a second end portion. The first end portion is disposed adjacent the base. A holder portion extends from the second end portion of the support portion. The holder portion defines an opening and is configured to be moved between a first configuration in which the opening defined by the holder portion has a first size and is configured to movably receive an interventional tool therethrough, and a second configuration in which the opening defined by the holder portion has a second size different than the first size.

Document CN 104 546 078 discloses a clinical puncture guidance positioning and fixing device. The clinical puncture guidance positioning and fixing device comprises an annular suction type suction cup, a supporting disk, a rotating disk and an arc-shaped arm, wherein the annular suction type suction cup consists of an annular top, an annular bottom, an outer side wall and an inner side wall; an adsorption ring groove of which the lower end is open is formed by the annular top, the outer side wall and the inner side wall together; an inner hole in the annular bottom is a puncture hole; a main body of the supporting disk is annular; the supporting disk is fixedly arranged on the upper side of the annular top; the rotating disk is annular and is supported by the supporting disk; the supporting disk and the rotating disk are coaxial with each other and can rotate relatively; a shaft seat is arranged on the upper end face of the rotating disk; a rotating shaft is arranged in the shaft seat; one end of the arc-shaped arm is supported by the rotating shaft, and a rotationally connected guidance sleeve is arranged at the other end of the arc-shaped arm.

Document WO 02/36032 discloses a breast stabilizing and support device that has a generally oval base with a central opening therethrough, a variable diameter cord or band positioned concentrically within the central opening and adapted to be slid over the breast and tightened at the breast base that are adapted to be attached to and adjustably positioned around the base for holding various instruments. The base is semirigid and compliant to form around the contours of the torso adjacent to the breast. Attached to the base and lying in the central opening is a loop of cord that is adjustable in diameter and adapted to surround and apply a radially inward, compressive force to the breast. One or more articulated arms have selectively lockable clamps at one end for securing the arms to the base at various positions. At another end the arms have attachment means such as jaws or clamps for securing one or more tools to the base in position to perform operations on the breast tissue.

Document US 2017/014200 discloses a medical support device for holding and positioning a needle. This device comprises two rotational elements and at least one needle guide attached to a rotational element. The needle guide guides the direction of insertion of a needle-like instrument and includes a guide portion that guides a needle or other needle-like instrument where the puncture point of the needle in a first position is different from the puncture point when the needle guide guides the needle in a second position.

Document US 2017/311978 discloses an apparatus for guiding a surgical needle with improved accuracy. The apparatus having a base for positioning the apparatus on a patient; a second arc member attached to the base; a first arc member moveably attached to the second arc member; an arm attached to a needle guide support at one end and moveably attached to the first arc member at a distal end, and an angle marking device attached to the arm to indicate a vertical reference point for measuring the angle of tilt of the arm from the vertical reference point relative to the base. Wherein the first arc member is configured to move on the second arc member to facilitate movement of the needle guide support in a cranio-caudal plane and the arm is configured to move on the first arc member to facilitate movement of the needle guide support in an axial plane.

Document US 2012/265098 discloses a needle stand that includes a base, an arching boom, and a tube, wherein the needle stand supports a biopsy needle. The base defines a plane and the base is configured to attach to a patient. The boom is attached to the base and has a length and a convex curvature and defines a plurality of holes along all or a portion of the length. The tube is sized to retain a needle handle of the biopsy needle. Each of the plurality of holes is sized to retain the tube and each of the plurality of holes defines an axis. The plane and each of the axes form a unique point of entry angle for the biopsy needle.

Document US 5,575,798 discloses a stereotactic device adaptable to align and orient a variety of medical devices, such as differently sized needles, cannulas, and guide wires, into the human body for procedures, such as tumor biopsies, percutaneous discectomies, cyst aspirations, and tumor localizations, is described. The use in a stereotactic device is adapted for use in combination with a stereotactic bridge which includes a span rotatable on its horizontal axis and movably affixed to a C.T. scan table or an X-ray table by a positioning mechanism that provides for both vertical and horizontal movement of the span. The stereotactic device interacts with a C.T. scanner to achieve placement of the selected medical device.

Therefore, there is a need to overcome the drawbacks of the background art.

### DISCLOSURE OF THE INVENTION

One object of the present invention is to provide a stand for a puncturing device that is easy and quick to mount and dismount on a puncturing site.

A further object of the present invention is to provide a stand for a puncturing device that allows using other puncturing devices, either supported by other stands or not, in the same puncturing area, for example at the same puncturing site.

The invention is disclosed in claim 1, the dependent claims defining preferred aspects.

### BRIEF DESCRIPTION OF THE DRAWINGS

Embodiments will now be described by way of example only, with reference to the Figures, in which:
Figure 1 is a perspective and side solid view of a stand in accordance with an embodiment of the invention positioned on a patient's skin and receiving a puncturing device.
Figure 2 is a cross-sectional view of a stand in accordance with an embodiment of the invention positioned on a patient's skin and receiving a puncturing device inserted into a patient's body and aiming a targeting site.
Figure 3 is a perspective and side solid view of a stand in accordance with an embodiment of the present disclosure.
Figure 4 is a perspective and side solid view of a stand in accordance with an embodiment of the present disclosure.
Figure 5 is a perspective and side solid view of a stand in accordance with an embodiment of the present disclosure.
Figure 6 is a perspective and side solid views of a stand in accordance with an embodiment of the present disclosure.
Figure 7 is a perspective and side solid views of a stand in accordance with an embodiment of the present disclosure.
Figure 8 is a perspective and side solid views of a stand in accordance with an embodiment of the present disclosure.
Figure 9 is another perspective and side solid views of the stand of figure 8.
Figure 10 is another perspective and side solid views of the stand of figure 8.

### DETAILED DESCRIPTION

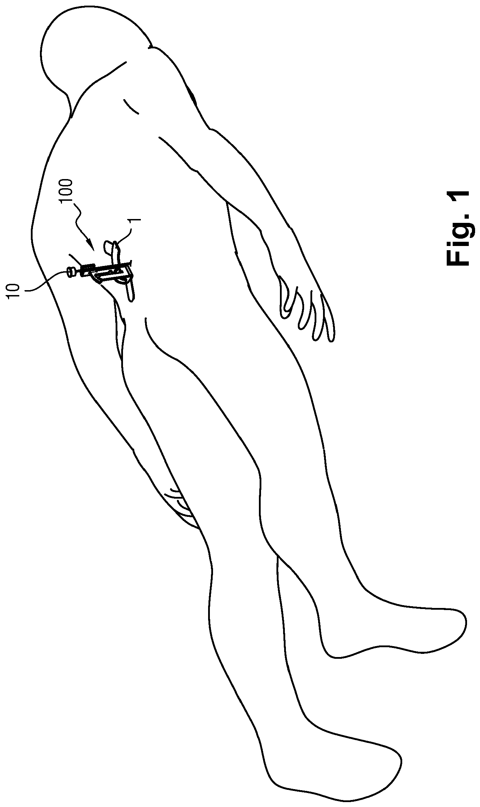

Figure 1 illustrates a stand 1 receiving a puncturing device 10 and positioned on a patient's skin 100.

Examples of puncturing devices are puncturing needles, anesthetic needles, biopsy guns, needle trocar, etc. Puncturing devices are often used during percutaneous interventions, wherein a target site inside a patient's body must be reached, e.g. in order to inject a product into the target site, withdraw material from the target site, or even destroy material of the target site. The stand 1 may be adapted to any kind of puncturing devices and also to any dimensions (e.g. diameter in the example of a puncturing needle) of a particular puncturing device.

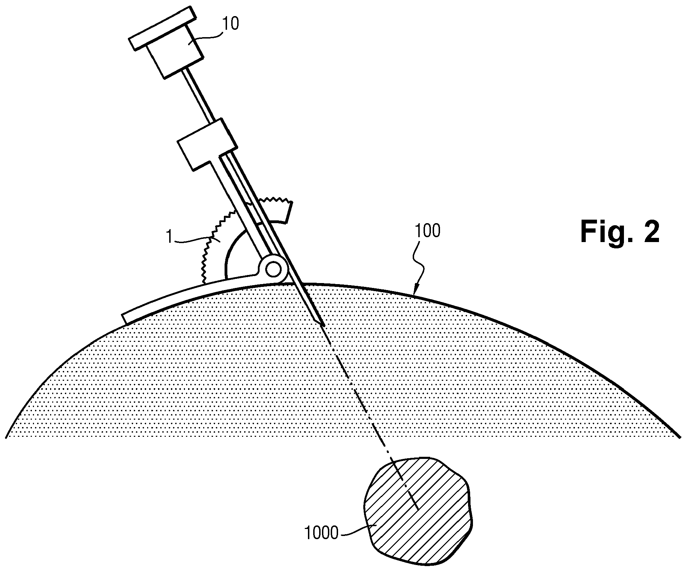

Figure 2 illustrates a puncturing device 10 received by the stand 1, the puncturing device 10 being partially inserted into the patient's body, through the patient's skin 100, and aiming at a target site 1000.

When a puncturing operation is performed, one may wish to determine accurately the puncturing position, along with the direction and the depth of the puncturing device 10. To this end, imaging systems such as Magnetic resonance imaging (MRI), Computerized tomography (CT), or X-rays systems are often used to provide images of a patient's internal organs.

Imaging of the patient's body may be performed before the puncturing device 10 is inserted through the patient's skin 100 or after the puncturing device 10 has been inserted into the patient's body. If so, the puncturing device 10 needs to be maintained in position while the imaging is performed. The stand 1 may be useful in this regard.

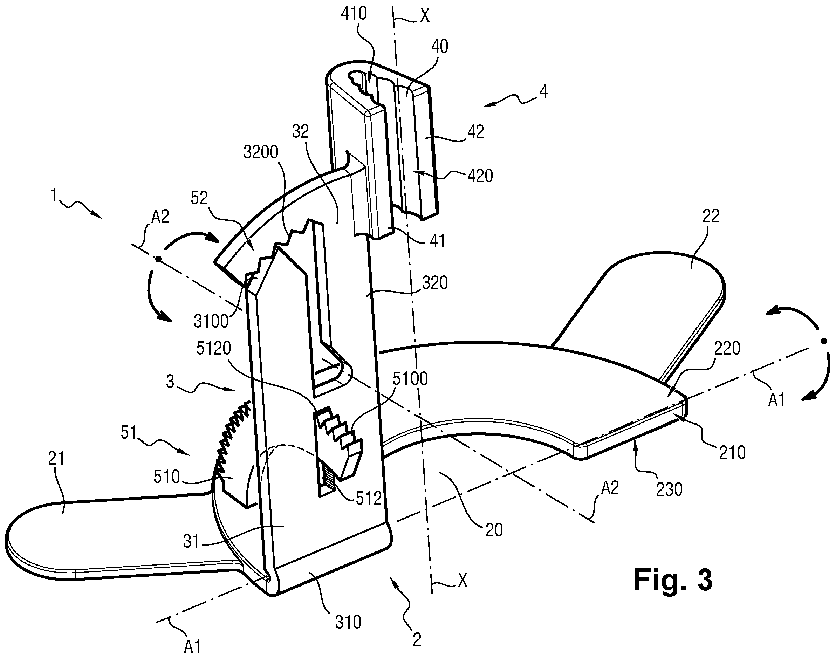

Figures 3 to 10 illustrate the stand 1 with more details.

The stand 1 comprises a base plate 2 adapted to be positioned on the patient's body, for example on the patient's skin 100. In the present text, the word "upper" and "lower" are used with reference to the patient's skin 100, an upper element being more distant from the patient's skin once the stand 1 is positioned on the patient's skin than a lower element.

To this end, the base plate 2 may be provided with at least one flat leg 21, 22, 23, 24 for positioning the stand 1 on the patient's skin 100. More precisely, the flat leg 21, 22, 23, 24 allows to increase the support surface of the base plate 2 over the patient's skin 100. This way, the puncturing device 10 may be as inclined as required with respect to a direction orthogonal to the patient's skin 100, without tilting.

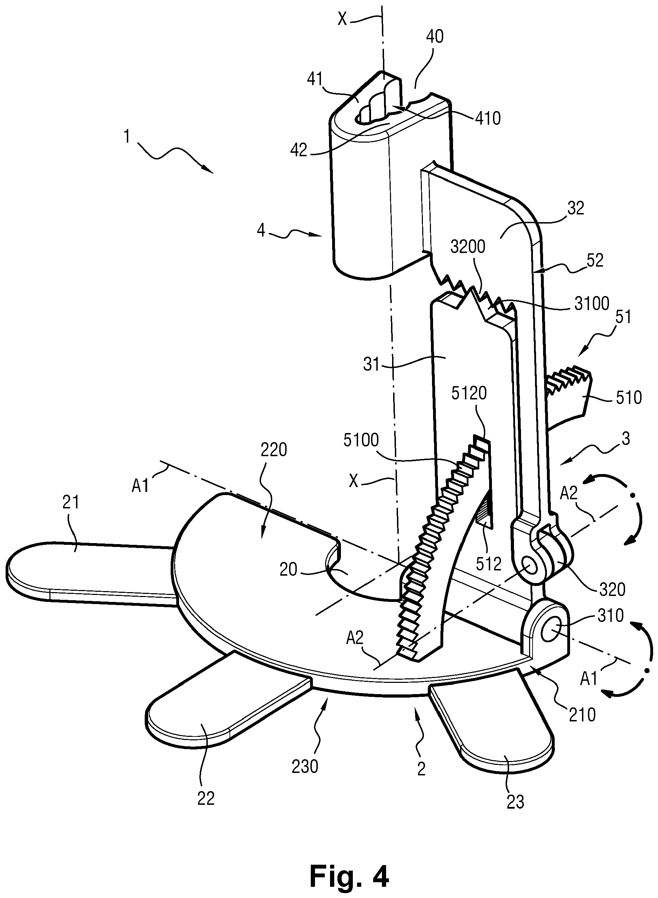

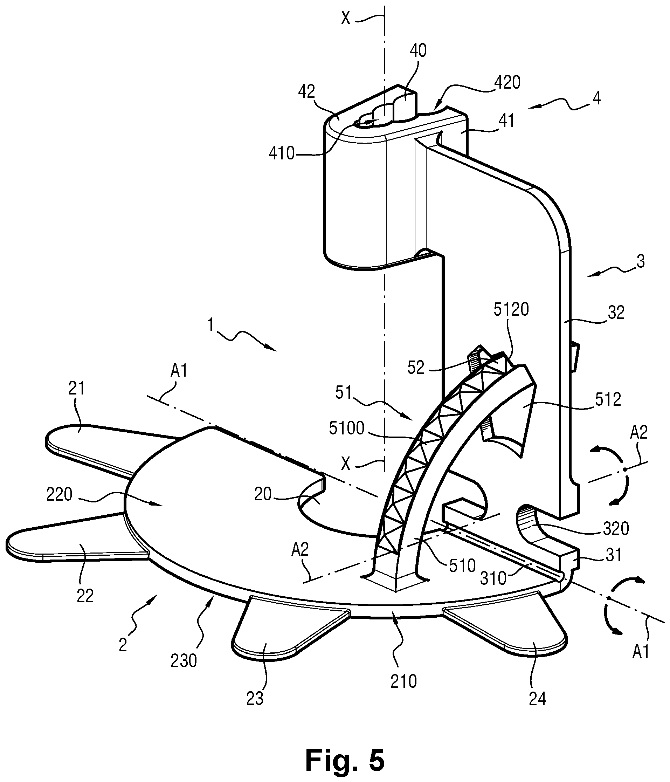

Figures 3, 6 and 7 illustrate a base plate 2 being provided with two flat legs 21, 22, whereas figure 4 illustrates a base plate 2 being provided with three flat legs 21, 22, 23 and figure 5 illustrates a base plate 2 being provided with four flat legs 21, 22, 23, 24.

Optionally, the flat leg 21, 22, 23, 24 is further configured to attach the stand 1 to the patient's skin 100, for example by being provided with a peelable adhesive suitable for attachment of the flat leg 21, 22, 23, 24 to the patient's skin 100.

Obviously, the flat leg 21, 22, 23, 24 may present various dimensions (e.g. length and/or width) and/or shape. Figures 3 to 7 are merely illustrative in this regard.

In some embodiments, the flat leg 21, 22, 23, 24 may be rigid and, in other embodiments, the flat leg 21, 22, 23, 24 may be flexible. More precisely, the flat leg 21, 22, 23, 24 is conformable with the patient's skin 100. In other words, the flat leg 21, 22, 23, 24 is adapted to position the stand 1 on the patient's skin 100 whatever the limb of the body. Therefore, the flat leg 21, 22, 23, 24 would be rigid when the stand 1 is positioned on the patient's back, whereas the flat leg 21, 22, 23, 24 would be flexible when positioned on the patient's shin.

In the embodiments illustrated in figures 3 to 7, the base plate 2 presents a first surface 210, a second surface 220 and a third surface 230. The first surface 210 intersects the second surface 220 and the third surface 230. As can be seen on these figures, the second surface 220 and the third surface 230 are two opposite main surfaces of the base plate 2. Also, the third surface 230 is adapted to be placed in contact with the patient's skin 100. Last; the first surface 210 forms an edge of the base plate 2 connecting the second surface 220 and the third surface 203. Besides, an opening is arranged into the first surface 210, the second surface 220 and the third surface 230, the second opening being arranged to removably receive the puncturing device 10. This helps the puncturing device 10 to be easily attached to or withdrawn from the stand 1. Also, the stand 1 and puncturing device 10 may be engaged in a more flexible way. Indeed, either the stand 1 may be positioned on the patient's skin 100 after the puncturing having penetrated into the patient's body or the puncturing device 10 may be first received by the stand 1 and the stand may then be positioned on the patient's skin 100.

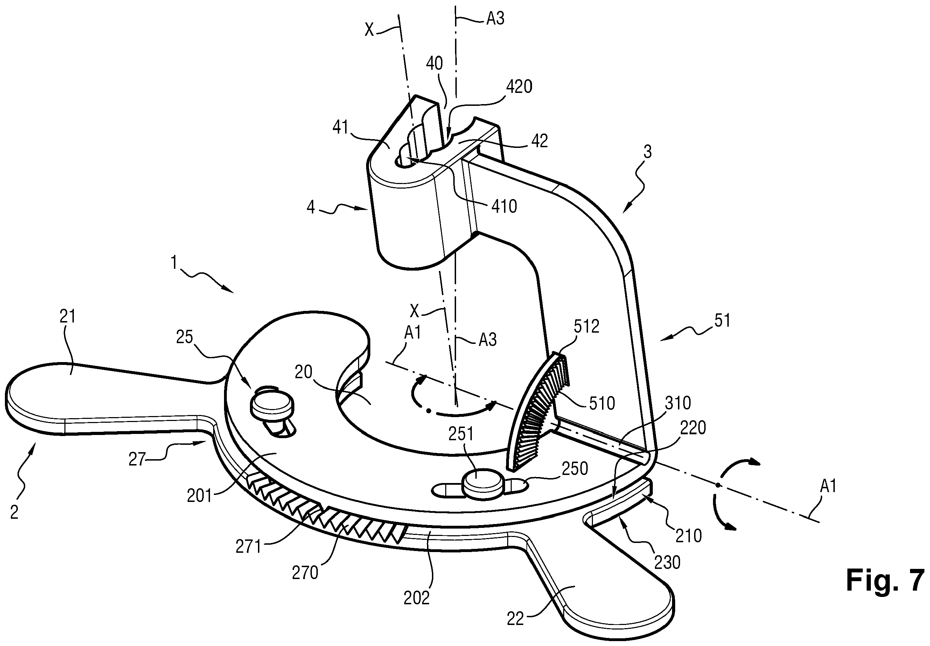

In one embodiment illustrated in figure 7, the base plate 2 comprises an upper portion 201 and a lower portion 202. In this embodiment, the arm 3 is pivotably connected to the upper portion 201 while the lower portion 202 is adapted to be positioned on the patient's body, and more precisely on the patient's skin 100. Also, the two flat legs 21, 22 are integral with the lower portion 202. As can be seen on figure 7, the upper portion 201 is pivotably connected to the lower portion 202. In the embodiment illustrated in figure 7, the upper portion 201 is pivotably connected to the lower portion 202 about a third axis A3 that is perpendicular to the patient's skin 100 when the base plate 2 is positioned on the patient's skin 100. In alternative embodiments, the upper portion 201 is pivotably connected to the lower portion about another axis, for example about the first axis or the second axis illustrated in figure 5. The provision of the upper portion 201 and the lower portion 202 pivotably connected to one another helps orienting the puncturing device 10 in any desirable position.

As can be seen on figure 7, the base plate 2 advantageously comprises a guiding mechanism 25. In this embodiment, the pivoting of the upper portion 201 and the lower portion 202 relative to one another is set by the guiding mechanism 25. To this end, as can be seen on figure 7, the guiding mechanism may comprise a guiding opening 250 cooperating with at least one pin 251. Figure 7 illustrates an embodiment wherein two guiding openings 250 cooperate with two pins 25, the guiding openings 250 being arranged within the upper portion 201 and the pins protruding from the lower portion 202. Of course, the guiding opening 250 may also be arranged within the lower portion 202 while the pin 25 protrude from the upper portion 201.

As can also be seen on figure 7, in one embodiment, the upper portion 201 is lockable to the lower portion 202 by a locking mechanism 27. The locking mechanism may comprise a plurality of teeth 270 arranged on a surface of one of the upper portion 201 or the lower portion 202, a protrusion 271 protruding from a corresponding surface of the other of the upper portion 201 or the lower portion 202. The protrusion 271 cooperate with the teeth 270 in order to lock the upper portion 201 relative to the lower portion 202. In the embodiment illustrated in figure 7, the teeth 270 are arranged on an upper surface of the lower portion 202 while the protrusion 271 is arranged on a lower surface of the upper portion 201. Of course, the teeth 270 may also be arranged on the lower surface of the upper portion 201 while the protrusion 271 is arranged on the upper surface of the lower portion 202.

The stand 1 further comprises an arm 3 pivotably connected to the base plate 2.

In the embodiments illustrated in figures 3 to 6, the arm 3 comprises a first portion 31 pivotably connected to the base plate 2 about a first axis A1, and a second portion 32 pivotably connected to the first portion 31 about a second axis A2. As can be seen from the figures, the first axis A1 is non-parallel to the second axis A2. This includes the following situations:
- the first axis A1 belongs to a first plane and the second axis A2 belongs to a second plane, the first plane being different from the second plane, the first plane may be parallel to the second plane, or
- the first axis A1 belongs to the same plane as the second axis A2, and the first axis A1 is not parallel to the second axis A2.

This allows the puncturing device 10 to be oriented in any suitable direction before and after having penetrated the patient's body while it is maintained by the stand 1. This may be useful when the skin suppleness needs to be taken into account by the user performing a percutaneous operation. Preferably, the first axis A1 is perpendicular to the second axis A2 so as to make it easier for the user to reach a targeted orientation. This means that, if the first axis A1 belongs to a first plane that is different to the second plane to which the second axis A2 belongs, then a projection of the first axis A1 on the second plane is perpendicular to the second axis A2.

Figures 3, 5 and 7 illustrate embodiments wherein the stand 1 comprises a first flexible hinge 310 integral with the first portion 31 of the arm 3 and the base plate 2, the first portion 31 of the arm 3 being pivotably connected to the base plate 2 by the first flexible hinge 310.

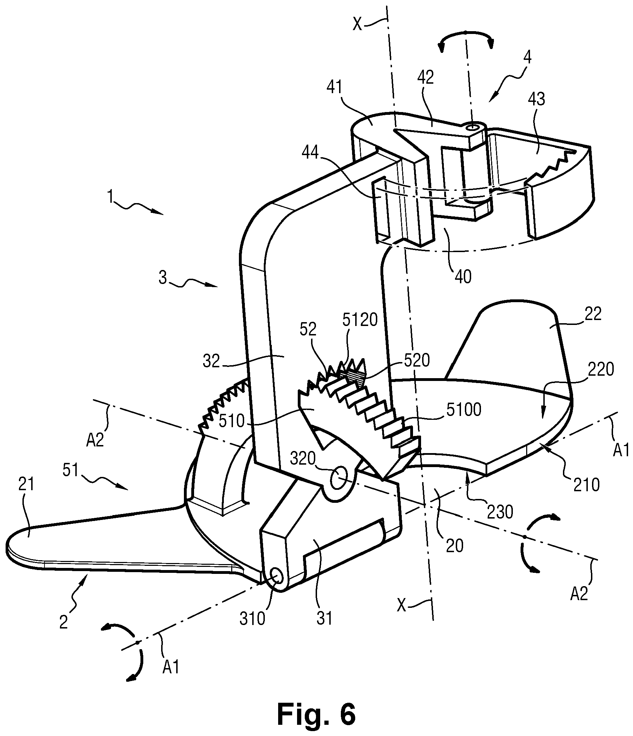

Figures 4 and 6 illustrate embodiments wherein the stand 1 comprises a first pivot hinge 310, the first portion 31 of the arm 3 being pivotably connected to the base plate 2 by the first pivot hinge 310.

Figures 3 and 5 illustrate embodiments wherein the stand 1 comprises a second flexible hinge 320 integral with the first portion 31 of the arm 3 and the second portion 32 of the arm 3, the second portion 32 of the arm 3 being pivotably connected to the first portion 31 of the arm 3 by the second flexible hinge 320.

Figures 4 and 6 illustrate embodiments wherein the stand 1 comprises a second pivot hinge 320, the second portion 32 of the arm 3 being pivotably connected to the first portion 31 of the arm 3 by the second pivot hinge 320.

Flexible hinges 310, 320 may consist in thinner portions, as illustrated in figures 3 and 5. The thinner portions may also be arcuate as in the first flexible hinge 310 illustrated in figures 3 and 5 or as in the second flexible hinge 320 illustrated in figure 5. Other examples of flexible hinges 310, 320 may also be used, such as with portions made out of at least one material that is more flexible than the material of the rest of the stand 1.

Embodiments wherein the stand 1 comprises a flexible hinge may be advantageous for this reduces the number of pieces needed to build the stand 1 and also reduces operating clearances when the stand 1 is manipulated.

As can be seen from figures 4 and 6, pivot hinges 310, 320 may comprise a pin inserted through at least one opening made into the base plate 2, the first portion 31 of the arm 3 or the second portion 32 of the arm 3. Advantageously, the at least one opening may be made into protrusions, for example:
- one opening made into each of two arcuate protrusions of the base plate 2 as in the first pivot hinge 310 illustrated in figure 4, or
- one opening made into one arcuate protrusion of the second portion 32 of the arm 3, as in the second pivot hinge 320 illustrated in figure 4, or
- one opening made into each of two arcuate protrusions of the first portion 31 of the arm 3, as in the second pivot hinge 320 illustrated in figure 4, or
- one opening made longitudinally within and along a cylindrical protrusion of the first portion 31 of the arm 3, as in the first pivot hinge 310 illustrated in figure 4, or made longitudinally within and along a cylindrical protrusion of the base plate 2, as in the first pivot hinge 310 illustrated in figure 6, or
- one opening made into each of two tabs protruding from the first portion 31 of the arm 3, as in the first pivot hinge 310 illustrated in figure 6, or made into each of two tabs protruding from the second portion 32 of the arm 3 as in the second pivot hinge 320 illustrated in figure 6.

In embodiments wherein the stand 1 comprises both a first pivot hinge 310 and a second pivot hinge 320, the second pivot hinge 320 may be located distant from the first pivot hinge 310 along the first portion 31 of the arm 3, as illustrated in figures 4 and 6. However, the second pivot hinge 320 may also be located closer to the first pivot hinge 310 and even belong to the same plane as the first pivot hinge 310 so that the first axis A1 and the second axis A2 belong to the same plane.

In an embodiment the first portion 31 may pivot around the first axis A1 within a range lying from -90° to 90°. And, in an embodiment, the second portion 32 may pivot around the second axis A2 within a range lying from -45° to 45°. This allows a huge flexibility for the user trying to adjust the orientation of the puncturing device 10 before or after the puncturing device 10 having been inserted into a patient's body.

As illustrated in figures 3 to 7, the stand 1 comprises a first positioning mechanism 51. The pivoting of the arm 3 and the base plate 2 relative to one another is set by the first positioning mechanism 51. The arm is lockable to the base plate 2 by the first positioning mechanism 51.

As can be seen in figures 3 to 7, the first positioning mechanism 51 comprises a rack 510 connected to the base plate 2, the rack 510 being arcuate in order to set the pivoting of the arm 3 relative to the base plate 2. For example, the arcuate rack 510 forms a portion of a ring section. The arcuate rack 510 is further adapted to penetrate into an opening 512 made through the arm 3 while the arm is pivoted relative to the base plate 2. This way the pivoting of the arm 3 and the base plate 2 relative to one another may be set.

In one embodiment illustrated in figures 3 to 6, the arcuate rack 510 comprises teeth 5100 that are formed on at least one surface of the arcuate rack 510, for example on an upper surface of the arcuate rack 510. Each one of the teeth 5100 is further adapted to cooperate with an edge 5120 of the opening 512 in order to lock the arm 3 relative to the base plate 2.

In an advantageous embodiment illustrated in figures 5 and 6, the pivoting of the first portion 31 and the second portion 32 relative to one another is also set by the first positioning mechanism 51. Also, the first portion 31 is lockable to the second portion 32 by the first positing mechanism. As can be seen from figures 5 and 6, this may be done by the edge 5120 being further serrated and the teeth 5100 being formed with a pyram idal-shape.

In another advantageous embodiment illustrated in figures 3 and 4, the stand 1 further comprises a second positioning mechanism 52. The pivoting of the first portion 31 and the second portion 32 relative to one another is the set by the second positioning mechanism 52. Also, the first portion 31 is lockable to the second portion 32 by the second positioning mechanism 52. As can be seen in figures 3 and 4, this may be done by the first portion 31 comprising a pointed tip 3100 and the second portion 32 comprising teeth 3200, the pointed tip 3100 cooperating with the teeth 3200.

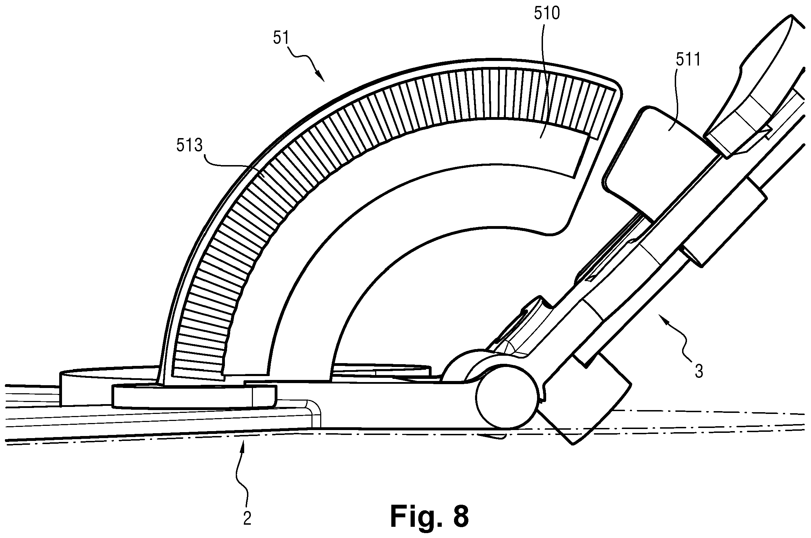

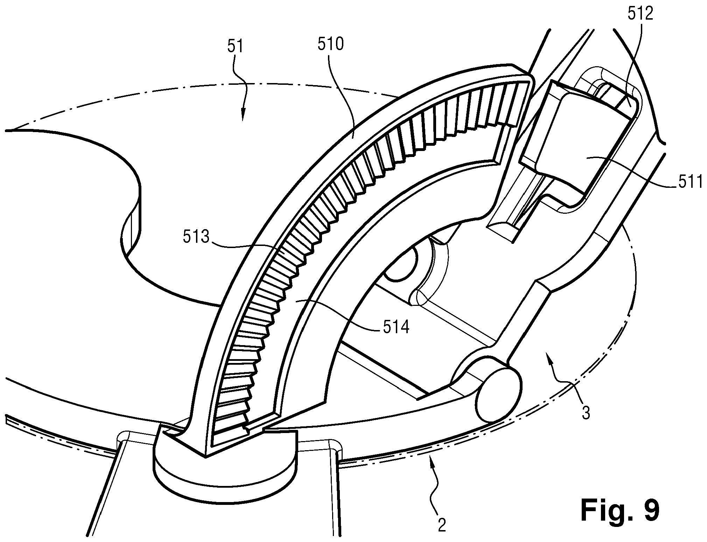

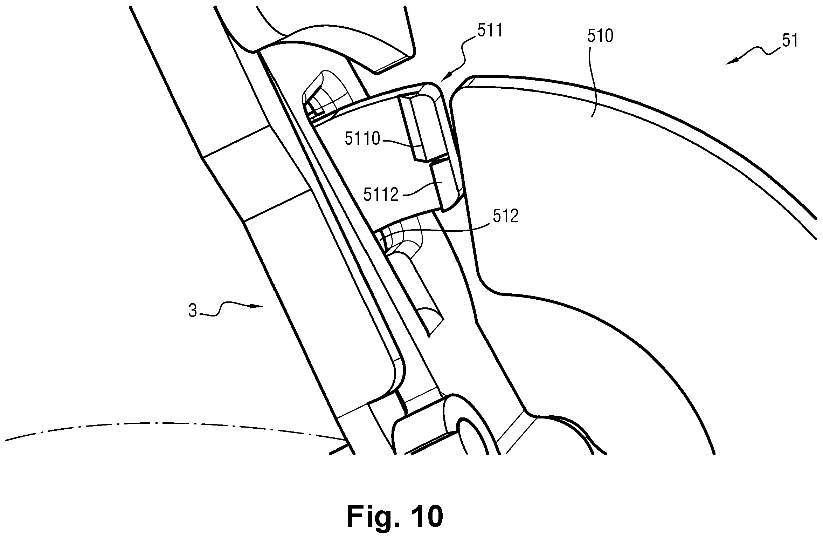

In another embodiment illustrated in figures 8 to 10, the first positioning mechanism 51 comprises a head 511 integral with and pivotably connected to the arm 3. The arcuate rack 510 comprises notches 513 formed on at least one surface of the arcuate rack 510, for example a side surface of the arcuate rack 510, as can be seen in figures 8 and 9. Each one of the notches 513 is then adapted to cooperate with the head 511 in order to lock the arm 3 relative to the base plate 2. Advantageously, as can be seen in figures 9 and 10, the arcuate rack 510 further comprises a groove 514 and the head 511 comprises two distinct edges 5110, 5112, the first edge 5110 being adapted to cooperate with the notches 513 in order to lock the arm 3 relative to the base plate 2, while the second edge 5112 being adapted to cooperate with the groove 514 in order to guide the pivoting movement of the arm 3 relative to the base plate 2. In this embodiment, the force required to pivot and lock the arm 3 relative to the base plate is less important than in the embodiments illustrated in figures 3 to 6. Indeed, the elastic return force of the head 511 is less than the elastic return force of the arcuate rack itself 510.

Other positioning mechanisms may be used in order to lock the second portion 32 to the first portion 31, such as a rail connection with a clamping collar.

Besides, the stand 1 comprises a guide 4 connected to the arm 3, for example by being integral to the arm 3, figures 3 to 6 illustrating the guide 4 being integral to the second portion 32 of the arm 3. As can be seen from figures 3 to 7, the guide has a longitudinal axis X-X. Also, the guide 4 comprises a groove 40 extending along the longitudinal axis X-X. The groove 40 is configured for engaging a portion of the puncturing device 10 according to a translation in a direction perpendicular to the longitudinal axis X-X in order to removably receive the puncturing device 10. This may help the stand 1 to be positioned in order to maintain the puncturing device 10 after the puncturing device 10 has penetrated into the patient's body. Of course, the groove 40 is also configured for engaging a portion of the puncturing device 10 according to a translation in a direction parallel to the longitudinal axis X-X in order to removably receive the puncturing device 10. This way, a puncturing device 10 may also be inserted into the groove 40 after the stand 1 has been secured to the patient's skin 100. This may be useful when several stands 1 and puncturing devices have already been set at the puncturing area.

In one embodiment, the guide 4 comprises a locking mechanism 41, 42, 410, 420, 43 configured to lock the puncturing device 10 in position once the groove 40 has engaged the portion of the puncturing device 10. This way, the puncturing device may be firmly fixed to the stand 1 and one has only to orient the arm 3 in order to orient the puncturing device 10.

Figures 3 to 7 illustrate several embodiments of the locking mechanism 41, 42, 410, 420, 43.

In the embodiments illustrated in figures 3 to 5, and 7, the guide 4 is a clamp 4 comprising two jaws 41, 42 extending along the longitudinal axis X-X, on both sides of the groove 40. In these embodiments, the jaws 41, 42 help the grove engaging a portion of the puncturing device 10 both according to a direction perpendicular and to a direction parallel to the longitudinal axis X-X. Also, the jaws 41, 42 help the stand 1 maintaining puncturing devices having various dimensions, such as needles having various diameters. In one embodiment, at least one of the jaws 41, 42 is adapted to elastically tend to close the groove 40 in order to lock the puncturing device 10 in position once the groove 40 has engaged the portion of the puncturing device 10. This helps maintaining the puncturing device 10 in position in a simple and practicable way.

Figures 3 to 5 and 7 illustrate embodiments wherein the groove 40 presents a serrated inner surface configured to lock the puncturing device 10 in position once the groove 40 has engaged the portion of the puncturing device 10. The serrated inner surface may further present various shapes and dimensions in order for the stand 1 to maintain puncturing devices with various dimensions, such as needles with various diameters.

Figure 6 illustrates an embodiment wherein the stand 1 further comprises a flap 43 articulated on the guide 4, for example on one of the two sides 41, 42 of the groove 40, and adapted to close the groove 40 once the groove 40 has engaged the portion of the puncturing device 10. As can be seen from this figure, the flap 43 may be configured to cooperate with an opening 44 of the stand 1, for example an opening 44 made into the second portion 32 of the arm 3, and may comprise teeth that cooperate with an edge of the opening in order to lock the flap 43 in position. This way, the groove 40 may be securely closed and the puncturing device 10 may be securely maintained into the groove 40. Also, the flap 43 may penetrate more or less deeply into the opening, which helps the stand 1 being adapted to maintain puncturing devices with various dimensions, such as needles with various diameters.

With the stand 1 as described, a puncturing device 10 may be engaged laterally so as to be maintained, for example during percutaneous interventions involving imaging steps. This way, several puncturing devices may be positioned at the same puncturing area since the stand 1 is not bulky. Also, the stand 1 may be easily displaced on the patient's skin 100, which make it easier for a user to adapt the puncturing area throughout the percutaneous intervention.

## Claims

1. A stand (1) for a puncturing device (10), the stand (1) comprising:
- a base plate (2) adapted to be positioned on a patient's body,
- an arm (3) pivotably connected to the base plate (2),
- a guide (4) connected to the arm (3) and having a longitudinal axis (X-X), the guide (4) further comprising a groove (40) extending along the longitudinal axis (X-X) and configured for engaging a portion of the puncturing device (10) according to a translation in a direction perpendicular to the longitudinal axis (X-X) in order to removably receive the puncturing device (10), wherein the arm (3) is lockable to the base plate (2) in order to maintain the puncturing device (10) in a determined orientation relative to the base plate (3), and
- a first positioning mechanism (51), wherein:
∘ the pivoting of the arm (3) and the base plate (2) relative to one another is set by the first positioning mechanism (51), and
∘ the arm (3) is lockable to the base plate (2) by the first positioning mechanism (51),
**characterized in that** the first positioning mechanism (51) comprises an arcuate rack (510) connected to the base plate (2), the arcuate rack (510) being adapted to penetrate into an opening (512) made through the arm (3) while the arm (3) is pivoted relative to the base plate (2) in order to set the pivoting of the arm (3) and the base plate (2) relative to one another.

2. A stand (1) according to claim 1, wherein the guide (4) comprises a locking mechanism (41, 42, 410, 420, 43) configured to lock the puncturing device (10) in position once the groove (40) has engaged the portion of the puncturing device (10).

3. A stand (1) according to claim 2, wherein the locking mechanism (41, 42, 410, 420, 43) comprises at least one of:
- the groove (40) presenting a serrated inner surface (410, 420),
- the guide (4) being a clamp comprising two jaws (41, 42) extending along the longitudinal axis (X-X), on both sides of the groove (40), at least one of the jaws (41, 42) being preferably adapted to elastically tend to close the groove (40), and
- a flap (43) articulated on the guide (4) and configured to close the groove (40).

4. A stand (1) according to one of claims 1 to 3, wherein the arm (3) comprises a first portion (31) pivotably connected to the base plate (2) about a first axis (A1), and a second portion (32) pivotably connected to the first portion (31) about a second axis (A2), the first axis (A1) being non-parallel to the second axis (A2), the first axis (A1) being preferably perpendicular to the second axis (A2).

5. A stand (1) according to one of claims 1 to 4, wherein the arcuate rack (510) comprises teeth (5100) formed on at least one surface of the arcuate rack (510), each one of the teeth (5100) being adapted to cooperate with an edge (5120) of the opening (512) in order to lock the arm (3) relative to the base plate (2).

6. A stand (1) according to one of claims 1 to 4, wherein:
- the first positioning mechanism (51) further comprises a head (511) integral with and pivotably connected to the arm (3), and
- the arcuate rack (510) comprises notches (513) formed on at least one surface of the arcuate rack (510), each one of the notches (513) being adapted to cooperate with the head (511) in order to lock the arm (3) relative to the base plate (2).

7. A stand (1) according to one of claims 1 to 5, wherein:
- the pivoting of the first portion (31) and the second portion (32) relative to one another is also set by the first positioning mechanism (51), and
- the first portion (31) is also lockable to the second portion (32) by the first positioning mechanism (51).

8. A stand (1) according to one of claims 4 to 6, further comprising a second positioning mechanism (52), wherein:
- the pivoting of the first portion (31) and the second portion (32) relative to one another is set by the second positioning mechanism (52), and
- the first portion (31) is lockable to the second portion (32) by the second positioning mechanism (52).

9. A stand (1) according to any of claims 1 to 8, wherein the base plate (2) presents a first surface (210), a second surface (220) and a third surface (230), the first surface (210) intersecting the second surface (220) and the third surface (230), an opening (20) being arranged into the first surface (210), the second surface (220) and the third surface (230), the opening (20) being arranged to removably receive the puncturing device (10).

10. A stand (1) according to any of claims 1 to 9, wherein the base plate (2) is provided with at least one flat leg (21, 22, 23, 24) for positioning the stand (1) on the patient's body.

11. A stand according to any of claims 1 to 10, wherein the base plate (2) comprises an upper portion (201) and a lower portion (202), wherein:
- the arm (3) is pivotably connected to the upper portion (201),
- the lower portion (202) is adapted to be positioned on the patient's body, and
- the upper portion (201) is pivotably connected to the lower portion (202).

12. A stand (1) according to claim 11, wherein the base plate (2) further comprises a guiding mechanism (25), the pivoting of the upper portion (201) and the lower portion (202) relative to one another being set by the guiding mechanism (25).

13. A stand (1) according to one of claims 11 and 12, wherein the base plate (2) further comprises a locking mechanism (27), the upper portion (201) being lockable to the lower portion (202) by the locking mechanism (27).

## Patentansprüche

1. Ständer (1) für eine Punktionsvorrichtung (10), wobei der Ständer (1) Folgendes umfasst:
- eine Basisplatte (2), die angepasst ist, an einem Körper eines Patienten positioniert zu werden,
- einen Arm (3), der schwenkbar mit der Basisplatte (2) verbunden ist,
- eine Führung (4), die mit dem Arm (3) verbunden ist und eine Längsachse (X-X) aufweist, wobei die Führung (4) ferner eine Rille (40) umfasst, die sich entlang der Längsachse (X-X) erstreckt und zum Ineinandergreifen mit einem Abschnitt der Punktionsvorrichtung (10) gemäß einer Translation in einer Richtung senkrecht zu der Längsachse (X-X) ausgestaltet ist, um die Punktionsvorrichtung (10) abnehmbar aufzunehmen, wobei der Arm (3) an der Basisplatte (2) verriegelbar ist, um die Punktionsvorrichtung (10) in einer bestimmten Ausrichtung in Bezug auf die Basisplatte (3) zu halten, und
- einen ersten Positionierungsmechanismus (51), wobei:
o das Schwenken des Arms (3) und der Basisplatte (2) in Bezug aufeinander durch den ersten Positionierungsmechanismus (51) eingestellt wird, und
o der Arm (3) durch den ersten Positionierungsmechanismus (51) an der Basisplatte (2) verriegelbar ist,
**dadurch gekennzeichnet, dass**
der erste Positionierungsmechanismus (51) eine bogenförmige Zahnstange (510) umfasst, die mit der Basisplatte (2) verbunden ist, wobei die bogenförmige Zahnstange (510) angepasst ist, um in eine Öffnung (512) einzudringen, die durch den Arm (3) hergestellt ist, während der Arm (3) in Bezug auf die Basisplatte (2) geschwenkt wird, um das Schwenken des Arms (3) und der Basisplatte (2) in Bezug aufeinander einzustellen.

2. Ständer (1) nach Anspruch 1, wobei die Führung (4) einen Verriegelungsmechanismus (41, 42, 410, 420, 43) umfasst, der dazu ausgestaltet ist, die Punktionsvorrichtung (10) an ihrer Position zu verriegeln, nachdem die Rille (40) mit dem Abschnitt der Punktionsvorrichtung (10) in Eingriff gebracht wurde.

3. Ständer (1) nach Anspruch 2, wobei der Verriegelungsmechanismus (41, 42, 410, 420, 43) mindestens eines von Folgendem umfasst:
- die Rille (40), die eine gezahnte Innenoberfläche (410, 420) aufweist,
- die Führung (4), die eine Klemme ist, die zwei Backen (41, 42) umfasst, die sich entlang der Längsachse (X-X) auf beiden Seiten der Rille (40) erstrecken, wobei mindestens eine der Backen (41, 42) vorzugsweise angepasst ist, um elastisch dazu zu neigen, die Rille (40) zu schließen, und
- eine Klappe (43), die gelenkig an der Führung (4) gelagert und dazu ausgestaltet ist, die Rille (40) zu schließen.

4. Ständer (1) nach einem der Ansprüche 1 bis 3, wobei der Arm (3) einen ersten Abschnitt (31), der um eine erste Achse (A1) schwenkbar mit der Basisplatte (2) verbunden ist, und einen zweiten Abschnitt (32) umfasst, der um eine zweite Achse (A2) schwenkbar mit dem ersten Abschnitt (31) verbunden ist, wobei die erste Achse (A1) nicht parallel zur zweiten Achse (A2) ist, wobei die erste Achse (A1) vorzugsweise senkrecht zur zweiten Achse (A2) ist.

5. Ständer (1) nach einem der Ansprüche 1 bis 4, wobei die bogenförmige Zahnstange (510) Zähne (5100) umfasst, die auf mindestens einer Oberfläche der bogenförmigen Zahnstange (510) gebildet sind, wobei jeder einzelne der Zähne (5100) angepasst ist, um mit einem Rand (5120) der Öffnung (512) zusammenzuwirken, um den Arm (3) in Bezug auf die Basisplatte (2) zu verriegeln.

6. Ständer (1) nach einem der Ansprüche 1 bis 4, wobei:
- der erste Positionierungsmechanismus (51) ferner einen Kopf (511) umfasst, der einteilig mit dem Arm (3) und schwenkbar damit verbunden ist, und
- die bogenförmige Zahnstange (510) Kerben (513) umfasst, die auf mindestens einer Oberfläche der bogenförmigen Zahnstange (510) gebildet sind, wobei jede einzelne der Kerben (513) angepasst ist, um mit dem Kopf (511) zusammenzuwirken, um den Arm (3) in Bezug auf die Basisplatte (2) zu verriegeln.

7. Ständer (1) nach einem der Ansprüche 1 bis 5, wobei:
- das Schwenken des ersten Abschnitts (31) und des zweiten Abschnitts (32) in Bezug aufeinander auch durch den ersten Positionierungsmechanismus (51) eingestellt wird, und
- der erste Abschnitt (31) auch durch den ersten Positionierungsmechanismus (51) an dem zweiten Abschnitt (32) verriegelbar ist.

8. Ständer (1) nach einem der Ansprüche 4 bis 6, der ferner einen zweiten Positionierungsmechanismus (52) umfasst, wobei:
- das Schwenken des ersten Abschnitts (31) und des zweiten Abschnitts (32) in Bezug aufeinander durch den zweiten Positionierungsmechanismus (52) eingestellt wird, und
- der erste Abschnitt (31) durch den zweiten Positionierungsmechanismus (52) an dem zweiten Abschnitt (32) verriegelbar ist.

9. Ständer (1) nach einem der Ansprüche 1 bis 8, wobei die Basisplatte (2) eine erste Oberfläche (210), eine zweite Oberfläche (220) und eine dritte Oberfläche (230) aufweist, wobei die erste Oberfläche (210) die zweite Oberfläche (220) und die dritte Oberfläche (230) schneidet, wobei eine Öffnung (20) in der ersten Oberfläche (210), der zweiten Oberfläche (220) und der dritten Oberfläche (230) angeordnet ist, wobei die Öffnung (20) angeordnet ist, um die Punktionsvorrichtung (10) abnehmbar aufzunehmen.

10. Ständer (1) nach einem der Ansprüche 1 bis 9, wobei die Basisplatte (2) mit mindestens einem flachen Schenkel (21, 22, 23, 24) zum Positionieren des Ständers (1) auf dem Körper des Patienten versehen ist.

11. Ständer nach einem der Ansprüche 1 bis 10, wobei die Basisplatte (2) einen oberen Abschnitt (201) und einen unteren Abschnitt (202) umfasst, wobei:
- der Arm (3) schwenkbar mit dem oberen Abschnitt (201) verbunden ist,
- der untere Abschnitt (202) angepasst ist, um auf dem Körper des Patienten positioniert zu werden, und
- der obere Abschnitt (201) schwenkbar mit dem unteren Abschnitt (202) verbunden ist.

12. Ständer (1) nach Anspruch 11, wobei die Basisplatte (2) ferner einen Führungsmechanismus (25) umfasst, wobei das Schwenken des oberen Abschnitts (201) und des unteren Abschnitts (202) in Bezug aufeinander durch den Führungsmechanismus (25) eingestellt wird.

13. Ständer (1) nach einem der Ansprüche 11 und 12, wobei die Basisplatte (2) ferner einen Verriegelungsmechanismus (27) umfasst, wobei der obere Abschnitt (201) durch den Verriegelungsmechanismus (27) an dem unteren Abschnitt (202) verriegelbar ist.

## Revendications

1. Support (1) pour un dispositif de perforation (10), le support (1) comprenant :
- une plaque de base (2) adaptée pour être placée sur le corps d'un patient,
- un bras (3) relié de manière pivotante à la plaque de base (2),
- un guide (4) relié au bras (3) et présentant un axe longitudinal (X-X), le guide (4) comprenant en outre une gorge (40) s'étendant le long de l'axe longitudinal (X-X) et configurée pour engager une partie du dispositif de perforation (10) selon une translation dans une direction perpendiculaire à l'axe longitudinal (X-X) afin de recevoir de manière amovible le dispositif de perforation (10), dans lequel le bras (3) peut être verrouillé à la plaque de base (2) afin de maintenir le dispositif de perforation (10) dans une orientation déterminée par rapport à la plaque de base (3), et
- un premier mécanisme de positionnement (51), dans lequel :
∘ le pivotement du bras (3) et de la plaque de base (2) l'un par rapport à l'autre est réglé par le premier mécanisme de positionnement (51), et
∘ le bras (3) peut être verrouillé à la plaque de base (2) par le premier mécanisme de positionnement (51),
**caractérisé en ce que** le premier mécanisme de positionnement (51) comprend une crémaillère arquée (510) reliée à la plaque de base (2), la crémaillère arquée (510) étant adaptée pour pénétrer dans une ouverture (512) pratiquée à travers le bras (3) lorsque le bras (3) est pivoté par rapport à la plaque de base (2) afin de régler le pivotement du bras (3) et de la plaque de base (2) l'un par rapport à l'autre.

2. Support (1) selon la revendication 1, dans lequel le guide (4) comprend un mécanisme de verrouillage (41, 42, 410, 420, 43) configuré pour verrouiller le dispositif de perforation (10) en position une fois que la gorge (40) a engagé la partie du dispositif de perforation (10).

3. Support (1) selon la revendication 2, dans lequel le mécanisme de verrouillage (41, 42, 410, 420, 43) comprend au moins l'un des éléments suivants :
- la gorge (40) présente une surface intérieure dentelée (410, 420),
- le guide (4) est une pince comprenant deux mâchoires (41, 42) s'étendant le long de l'axe longitudinal (X-X), de part et d'autre de la gorge (40), au moins l'une des mâchoires (41, 42) étant de préférence adaptée pour tendre élastiquement à fermer la gorge (40), et
- un volet (43) articulé sur le guide (4) et configuré pour fermer la gorge (40).

4. Support (1) selon l'une des revendications 1 à 3, dans lequel le bras (3) comprend une première partie (31) reliée de manière pivotante à la plaque de base (2) autour d'un premier axe (A1), et une deuxième partie (32) reliée de manière pivotante à la première partie (31) autour d'un deuxième axe (A2), le premier axe (A1) n'étant pas parallèle au deuxième axe (A2), le premier axe (A1) étant de préférence perpendiculaire au deuxième axe (A2).

5. Support (1) selon l'une des revendications 1 à 4, dans lequel la crémaillère arquée (510) comprend des dents (5100) formées sur au moins une surface de la crémaillère arquée (510), chacune des dents (5100) étant adaptée pour coopérer avec un bord (5120) de l'ouverture (512) afin de verrouiller le bras (3) par rapport à la plaque de base (2).

6. Support (1) selon l'une des revendications 1 à 4, dans lequel :
- le premier mécanisme de positionnement (51) comprend en outre une tête (511) solidaire du bras (3) et reliée de manière pivotante à celui-ci, et
- la crémaillère arquée (510) comprend des encoches (513) formées sur au moins une surface de la crémaillère arquée (510), chacune des encoches (513) étant adaptée pour coopérer avec la tête (511) afin de verrouiller le bras (3) par rapport à la plaque de base (2).

7. Support (1) selon l'une des revendications 1 à 5, dans lequel :
- le pivotement de la première partie (31) et de la deuxième partie (32) l'une par rapport à l'autre est également réglé par le premier mécanisme de positionnement (51), et
- la première partie (31) peut également être verrouillée à la deuxième partie (32) par le premier mécanisme de positionnement (51).

8. Support (1) selon l'une des revendications 4 à 6, comprenant en outre un second mécanisme de positionnement (52), dans lequel :
- le pivotement de la première partie (31) et de la deuxième partie (32) l'une par rapport à l'autre est réglé par le deuxième mécanisme de positionnement (52), et
- la première partie (31) peut être verrouillée à la seconde partie (32) par le second mécanisme de positionnement (52).

9. Support (1) selon l'une des revendications 1 à 8, dans lequel la plaque de base (2) présente une première surface (210), une deuxième surface (220) et une troisième surface (230), la première surface (210) recoupant la deuxième surface (220) et la troisième surface (230), une ouverture (20) étant ménagée dans la première surface (210), la deuxième surface (220) et la troisième surface (230), l'ouverture (20) étant ménagée pour recevoir de manière amovible le dispositif de perforation (10).

10. Support (1) selon l'une des revendications 1 à 9, dans lequel la plaque de base (2) est pourvue d'au moins une patte plate (21, 22, 23, 24) pour positionner le support (1) sur le corps du patient.

11. Support selon l'une des revendications 1 à 10, dans lequel la plaque de base (2) comprend une partie supérieure (201) et une partie inférieure (202), dans laquelle :
- le bras (3) est relié de manière pivotante à la partie supérieure (201),
- la partie inférieure (202) est adaptée pour être positionnée sur le corps du patient, et
- la partie supérieure (201) est reliée de manière pivotante à la partie inférieure (202).

12. Support (1) selon la revendication 11, dans lequel la plaque de base (2) comprend en outre un mécanisme de guidage (25), le pivotement de la partie supérieure (201) et de la partie inférieure (202) l'une par rapport à l'autre étant réglé par le mécanisme de guidage (25).

13. Support (1) selon l'une des revendications 11 et 12, dans lequel la plaque de base (2) comprend en outre un mécanisme de verrouillage (27), la partie supérieure (201) pouvant être verrouillée à la partie inférieure (202) par le mécanisme de verrouillage (27).
